# EUROPEAN PATENT SPECIFICATION

(11) **EP 1 637 131 B1**
(45) Date of publication and mention of the grant of the patent: **21.10.2009**
(21) Application number: 04717783.7
(22) Date of filing: 05.03.2004
(51) Int. Cl.: A61K 9/70, A61K 47/02, A61K 47/10, A61K 47/32

(54) **CATAPLASM BASE AND CATAPLASM USING THE SAME**
CATAPLASMA-BASE UND DIESE VERWENDENDES CATAPLASMA
BASE DE CATAPLASME ET CATAPLASME L'UTILISANT

(30) Priority: 07.03.2003 JP 2003006733
(43) Date of publication of application: 22.03.2006
(73) Proprietor: TOAGOSEI CO., LTD., Minato-ku, Tokyo 105-8419 (JP); Nihon Junyaku Co., Ltd., Tokyo 103-0021 (JP)
(72) Inventor: ITO, Kenji, c/o Toagosei Co., Ltd, Minato-ku, Nagoya-shi, Aichi 455-0027 (JP); USUKI, Mizuyo, c/o Nihon Junyaku Co., Ltd, Futaba-gun Fukushima 979-0401 (JP); AIMIYA, Ryoichi, c/o Nihon Junyaku Co., Ltd, Futaba-gun Fukushima 979-0401 (JP)
(74) Representative: Ward, David Ian
(86) International application number: PCT/JP2004/002841
(87) International publication number: WO 2004/078165

(56) References cited:
- EP-A- 0 321 650
- EP-A2- 0 446 636
- WO-A-00/35961
- WO-A-01/97772
- WO-A-91/15250
- JP-A- 6 087 961
- JP-A- 2000 053 564
- US-A- 3 768 565
- US-A- 5 185 395
- DATABASE WPI Week 198611 Derwent Publications Ltd., London, GB; AN 1986-073070 XP002440850 & JP 61 023670 A (NISSHIN KAGAKU KOGYO KK) 1 February 1986 (1986-02-01)

## Description

### <Technical Field>

The present invention relates to a cataplasm base and a cataplasm including a backing composed of a non-woven fabric or the like on which paste including the base, drugs, moisture, and the like is applied. To be specific, the present invention relates to a cataplasm base having high moisture content, that can maintain a cooling effect for a long time, and having an excellent property in percutaneous absorption of drugs, adhesive strength, spreading and shape-retaining properties, and heat resistance; and a cataplasm including a backing having the base thereon.

### <Background Art>

Cataplasms including a backing composed of a non-woven fabric or the like on which paste including a base mainly composed of a hydrophilic or water-soluble polymer and water is spread, the paste further including a moisturizing agent, drugs, or the like, have been widely used and the application thereof has also been extended. Consequently, in order to improve the percutaneous absorption property of drugs and the continuity of cooling time, research and development of cataplasm bases having high moisture content has been conducted. In the present invention, the base refers to a substance that holds medicinal components.

As a hydrophilic cataplasm base, hydrophilic polymers such as polyvinyl alcohol, carboxymethylcellulose, polyacrylic acid (or a salt thereof), and the like are used. In general, such hydrophilic bases are safer for the skin and have excellent water retention, compared with nonaqueous bases, but the adhesive force is insufficient. The increase in adhesive force decreases the cohesive force of hydrophilic polymer. As a result, when the paste is detached from the skin, the paste easily remains on the skin.

In hydrophilic bases, instead of using known hydrophilic polymers, using a crosslinked hydrophilic polymer or adding a polyvalent metal salt to a hydrophilic polymer base to form intermolecular crosslink in order to improve adhesive strength or shape-retaining property of the base.

For example, as a base composed of a crosslinked polymer having a sulfonic acid group, Japanese Unexamined Patent Application Publication No. 4-91021 discloses a cataplasm including a crosslinked hydrophilic polymer prepared by copolymerizing 2-acrylamido-2-methylpropane sulfonic acid (or a salt thereof) and acrylic acid (or a salt thereof) in the presence of a polyfunctional crosslinking agent. Also, Japanese Unexamined Patent Application Publication No. 9-124466 discloses a crosslinked hydrophilic polymer prepared by performing a crosslinking polymerization of 2-acrylamido-2-methylpropane sulfonic acid (or a salt thereof), acrylic acid (or a salt thereof), and a crosslinkable monomer having at least two ethylenically unsaturated bonds.

In paste including such a crosslinked hydrophilic polymer, granular gel particles in the paste have a certain degree of shape-retaining property but each of the gel particles does not have fluidity. Therefore, the paste including these gel particles have a poor spreading property on a backing. Accordingly, it is difficult to produce a paste layer having a smooth finished surface. Furthermore, since each of the gel particles is completely crosslinked, the adhesive force of the paste is low. Consequently, in order to increase the adhesive force, when the crosslinking reaction performed after spreading on a backing is relieved, the shape-retaining property of the paste becomes insufficient. As a result, it is impossible to combine adhesive force and shape-retaining property. Such paste does not sufficiently function as a cataplasm.

In addition, paste including polyacrylic acid (or a salt thereof), a crosslinked highly water-absorbing polymer having a sulfonic acid group, and a polyvalent metal compound for the purpose of intermolecular crosslinking is disclosed. However, when the paste contains a large amount of the highly water-absorbing polymer having a sulfonic acid group, disadvantageously, the adhesive force is insufficient and the surface of the paste cannot be smoothed (Japanese Unexamined Patent Application Publication No. 2003-155252).

Methods for forming a sheet from a gel composition includes a method of mixing a hydrophilic polymer prepared by polymerizing a monomer component such as acrylic acid or 2-acrylamido-2-methylpropane sulfonic acid with an component such as a drug, and applying the mixture on a backing; and a method of blending a monomer of starting material of a hydrophilic polymer, a crosslinking agent, a drug, other additives, and the like in advance, casting the mixture on a backing or a frame, and polymerizing the mixture by, for example, ultraviolet irradiation.

For example, a highly adhesive hydrogel polymer is produced by copolymerizing 2-acrylamido-2-methylpropane sulfonic acid (or a salt thereof) and a crosslinkable monomer together with a polyhydric alcohol serving as a wetting agent by ultraviolet irradiation in an aqueous medium with a pH of 5.5 or more (Japanese Unexamined Patent Application Publication No. 6-200224). Furthermore, Japanese Unexamined Patent Application Publication No. 9-124465 discloses a method for producing a base for percutaneous absorption preparation, the method including photo polymerization of a composition including a monomer having an ethylenically unsaturated bond, a drug, and a photoinitiator with an active energy ray. Japanese Examined Patent Application Publication No. 5-502239 discloses a method of applying a solution on a film, the solution containing a monofunctional monomer such as 2-acrylamido-2-methylpropane sulfonic acid, acrylic acid, or the like; a crosslinkable monomer having at least two ethylenically unsaturated bonds; a moisturizing agent; a drug; and the like; and then polymerizing the monomers to cause gelation by ultraviolet irradiation.

In the above methods, when a hydrophilic polymer is prepared by polymerization, a paste layer is also formed at the same time. Therefore, these methods are advantageous in that a monomer solution having low viscosity can be easily prepared by mixing and a crosslinking reaction can be performed in a short period of time because photopolymerization is employed. However, drugs that are decomposed by ultraviolet rays cannot be used. In addition, a large amount of unreacted monomers easily remains on a paste layer prepared by such a direct polymerization method. Also, the purification of the paste layer itself is also difficult.

As described above, although the moisture content can be increased to some extent, known cataplasm bases have the following problems: The adhesive strength to the skin is poor and the paste may seep from a backing composed of a non-woven fabric or the like. In addition, when a cataplasm is detached from the skin, the paste remains on the skin and thus the paste cannot be cleanly detached. Thus, the known cataplasm bases are not satisfactory in view of adhesive strength to the skin, water retention, shape-retaining property, hardness, and the like.

### <Disclosure of Invention>

In order to solve the above problems, the present inventors have conducted extensive studies and found the following:
A gel composition includes
   (a) a water-soluble copolymer;
   (b) a polyhydric alcohol;
   (c) a polyvalent metal compound; and
   (d) water;
   wherein the water-soluble copolymer is in the form of fine powder and includes 1 to 30 mole percent of 2-acrylamido-2-methylpropane sulfonic acid and 70 to 99 mole percent of acrylic acid as monomer units, wherein 20 to 60 mole percent of the acids is neutralized, and wherein the content of unreacted monomers in the water-soluble copolymer is 0.5 weight percent or less;

And even when the gel composition contains 80 weight percent or more of moisture, a cooling effect can be maintained for a long time, the gel composition has an excellent percutaneous absorption property of drugs, adhesive strength, and shape-retaining property, the gel composition has an excellent spreading property to provide a smooth finished surface of a paste layer, and the paste layer has appropriate hardness and does not easily remain on the skin when detached from the skin. Based on these findings, the present invention has been made.

The present invention will now be described in detail.

### <Best Mode for Carrying Out the Invention>

### 1. Composition of cataplasm base

A cataplasm base of the present invention includes as indispensable components
(a) a water-soluble copolymer;
(b) a polyhydric alcohol;
(c) a polyvalent metal compound; and
(d) water.

### (a) Water-soluble copolymer

A water-soluble copolymer is produced by polymerizing a monomer mixture including 2-acrylamido-2-methylpropane sulfonic acid and/or a salt thereof and acrylic acid and/or a salt thereof, which form a polymer; or a monomer mixture including the above monomer mixture and another monomer optionally added, in the absence of a crosslinking agent.

2-acrylamido-2-methylpropane sulfonic acid and/or a salt thereof is used for providing paste with hardness to maintain the shape-retaining property. The amount used in the polymerization, that is, the amount substantially constituting the polymer is generally 1 to 30 mole percent based on the total number of moles of total monomer units constituting the water-soluble copolymer.

At a content of less than 1 mole percent, when an alcohol or a fatty acid is mixed in a base or when the base contains 80 weight percent or more of moisture, the shape-retaining property of the paste is insufficient and the paste seeps from a backing. Consequently, the hardness of the paste is decreased and the paste easily remains on the skin when detached.

On the other hand, at a content of more than 30 mole percent, although the paste is difficult to remain on the skin when detached, the adhesive force is decreased. Consequently, it is difficult to combine adhesive strength and shape-retaining property of the paste.

Examples of the salt of 2-acrylamido-2-methylpropane sulfonic acid include salts of an alkali metal such as sodium and potassium; ammonium salts; and salts of an organic amine such as triethylamine and triethanolamine. These salts can be readily prepared by neutralizing 2-acrylamido-2-methylpropane sulfonic acid with a corresponding alkali.

The content of acrylic acid and/or a salt thereof is generally 70 to 99 mole percent based on the total number of moles of total monomer units constituting the water-soluble copolymer as in the above-described content. At a content of less than 70 mole percent, it is difficult to combine adhesive strength and shape-retaining property. On the other hand, at a content of more than 99 mole percent, the shape-retaining property is insufficient and the paste seeps from a backing. Consequently, the hardness of the paste is decreased and the paste easily remains on the skin when detached.

Examples of the salt of acrylic acid include salts of an alkali metal such as sodium and potassium; ammonium salts; and salts of an organic amine such as triethylamine and triethanolamine. These salts can be readily prepared by neutralizing acrylic acid with a corresponding alkali.

The water-soluble copolymer, as described above, includes 2-acrylamido-2-methylpropane sulfonic acid and/or a salt thereof and acrylic acid and/or a salt thereof as indispensable monomer units. However, in the present invention, the water-soluble copolymer also includes copolymers containing these monomer units and another monomer to such an extent that does not impair the performance of the base (preferably, 10 mole percent or less relative to the total number of moles of total monomer units).

Examples of the other monomer include unsaturated carboxylic acids such as methacrylic acid, crotonic acid, itaconic acid, and maleic acid; alkali metal salts thereof; ammonium salts thereof; unsaturated sulfonic acids such as a (meth)acrylamido alkylalkane sulfonic acid other than 2-acrylamido-2-methylpropane sulfonic acid, allylsulfonic acid, methallyl sulfonic acid, styrene sulfonic acid, and vinyl sulfonic acid; alkali metal salts thereof; ammonium salts thereof; vinylpyrrolidinone; (meth)acrylamide; (meth)acrylonitrile; vinyl acetate; dimethylaminoethyl (meth)acrylate; hydroxyethyl (meth)acrylate; hydroxypropyl (meth)acrylate; polyethylene glycol mono(meth)acrylate; propylene glycol mono(meth)acrylate; methoxypolyethylene glycol mono(meth)acrylate; methyl (meth)acrylate; and ethyl (meth)acrylate. At least one of these can be used.

The neutralization ratio of water-soluble copolymer is 20 to 60 mole percent. A neutralization ratio of less than 20 mole percent is not preferable in view of skin irritation because the pH of paste is excessively decreased.

At a neutralization ratio of more than 60 mole percent, it is impossible to combine adhesive strength and shape-retaining property because of a decrease in adhesive force.

The neutralization ratio represents the ratio between 2-acrylamido-2-methylpropane sulfonic acid and acrylic acid; and salts thereof.

A water-soluble copolymer having a target neutralization ratio is prepared by polymerizing an acid and a salt serving as monomers or by neutralizing an acid during polymerization or the resultant acid polymer with an alkali.

The content of unreacted monomers remaining in the water-soluble copolymer is 0.5 weight percent or less of the total mass of the polymer. If the content of remaining unreacted monomers exceeds 0.5 weight percent, skin irritation is undesirably caused.

With respect to the molecular weight of the water-soluble copolymer, the water-soluble copolymer preferably has a weight-average molecular weight of 1,000,000 to 20,000,000 measured by aqueous gel permeation chromatography (hereinafter abbreviated as GPC) using polyethylene oxide as a standard material.

If the molecular weight is less than 1,000,000, the shape-retaining property is insufficient and paste seeps from a backing. Consequently, the hardness of the paste is decreased and the paste easily remains on the skin when detached. If the molecular weight exceeds 20,000,000, a product that is not dissolved in water is increased, thereby impairing water solubility of the polymer.

The water-soluble copolymer used in the present invention is in the form of fine powder. The solid content is preferably at least 90 weight percent. If the water-soluble copolymer has a solid content of less than 90 weight percent, the dispersion stability of polymer to polyhydric alcohols may be deteriorated.

With respect to the particle size distribution of fine powder, at least 90 weight percent of the particles preferably has a particle diameter of 180 µm or less, and in addition, particles having a diameter of 75 µm or less is preferably 80 weight percent or less based on the total mass of the powder.

If the amount of particles having a particle diameter of 180 µm or more exceeds 10 weight percent, it may be difficult to provide paste having a smooth finished surface. If the amount of particles having a particle diameter of 75 µm or less exceeds 80 weight percent, the molecular weight is easily decreased and workability may also be deteriorated.

The water-soluble copolymer can be synthesized by a known polymerization method. Specifically, examples of the method include gel polymerization, aqueous solution polymerization, and reversed-phase suspension polymerization.

Redox polymerization initiators are preferable as a polymerization initiator. Instead of using a redox polymerization initiator, radical polymerization may be performed by irradiating an active energy ray such as ultraviolet rays to a monomer aqueous solution containing a photoinitiator.

Examples of the polymerization initiator include persulfates of an alkali metal such as sodium persulfate and potassium persulfate; persulfates such as ammonium persulfate; organic peroxides such as hydrogen peroxide, cumene hydroperoxide, tert-butyl peroxide, and benzoyl peroxide; and azo compounds such as 2,2'-azobis(4-cyanovaleric acid), 2,2'-azobis[2-methyl-N-(2-hydroxyethyl)-propionamide], and 2,2'-azobisisobutyronitrile.

Furthermore, in polymerization, a reducing agent for forming a redox, for example, a transition metal salt, a hydrogensulfite, L-ascorbic acid (or a salt thereof), erythorbic acid (or a salt thereof), or an amine compound is preferably used in combination.

The amount of polymerization initiator added is adjusted according to the type of polymerization initiator used; the composition, the degree of polymerization, and the viscosity of the target polymer; and the like. The amount is generally 5 to 10,000 ppm, preferably 10 to 5,000 ppm, and particularly 15 to 3,000 ppm based on the total amount of total monomer.

The content of water-soluble copolymer in a base is generally 1 to 30 weight percent and preferably 3 to 15 weight percent based on the total amount of base. If the amount is less than 1 weight percent, the shape-retaining property and the hardness of the resultant base are decreased. Consequently, the paste may easily remain on the skin when detached from the skin. If the amount exceeds 30 weight percent, the balance between the hardness and the adhesive force of the resultant base may not be kept.

### (b) Polyhydric alcohol

The cataplasm base of the present invention includes a polyhydric alcohol as an indispensable component. This polyhydric alcohol serves as a moisturizing agent or a water retention agent.

Examples of the polyhydric alcohol preferably used include glycerin, sorbitol, mannitol, xylitol, ethylene glycol, diethylene glycol, 1,3-propanediol, 1,4-butanediol, polyethylene glycol, propylene glycol, and polypropylene glycol.

The amount is generally 1 to 50 weight percent and preferably 5 to 30 weight percent based on the total amount of base. If the amount is less than 1 weight percent, moisture may be vaporized from the resultant base, resulting in drying of the base. In such a case, drugs may be precipitated in the base. If the amount exceeds 50 weight percent, the balance between the hardness and the adhesive force of the resultant base may not be kept.

### (c) Polyvalent metal compound

The cataplasm base of the present invention includes a polyvalent metal compound as an indispensable component. This polyvalent metal compound releases a polyvalent metal ion. The released polyvalent metal ion forms an ionic bond with an anionic group (for example, carboxylic acid group or sulfonic acid group) of the water-soluble copolymer (a) to form a crosslinked structure. This crosslinked structure imparts characteristics such as adhesive strength, gel-formation capacity, high hydroscopic property, shape-retaining property, and hardness to the base.

Examples of the polyvalent metal compound include polyvalent metal compounds that release a polyvalent metal ion such as an aluminum ion, a magnesium ion, a calcium ion, a zinc ion, a cadmium ion, a titanium ion, a chromium ion, a manganese ion, and an iron ion.

Since the cataplasm base and the cataplasm of the present invention are used by applying them directly on the human skin or the like, the cataplasm base and the cataplasm must be safe. Accordingly, aluminum compounds, magnesium compounds, calcium compounds, or zinc compounds, which have excellent safety, are preferably used as the polyvalent metal compound.

Examples of the polyvalent metal compound preferably used include aluminum succinate, aluminum glycinate, aluminum acetate, aluminum hydroxide, aluminum sulfate, aluminum chloride, aluminum oxide, aluminum silicate, calcium hydroxide, calcium carbonate, calcium chloride, calcium sulfate, calcium nitrate, calcium phosphate, calcium hydrogenphosphate, calcium acetate, calcium oxide, magnesium hydroxide, magnesium carbonate, magnesium chloride, magnesium sulfate, magnesium nitrate, magnesium phosphate, magnesium acetate, magnesium oxide, magnesium silicate, aluminum magnesium hydroxide, magnesium aluminometasilicate, magnesium aluminosilicate, potassium alum, ammonium alum, and iron alum. At least one of these can be used.

Among these, aluminum hydroxide, aluminum glycinate, or aluminum chloride is preferable in view of high gel strength. In particular, aluminum glycinate or aluminum hydroxide is preferable.

The amount is generally 0.001 to 3 weight percent, preferably 0.01 to 1 weight percent, and more preferably 0.01 to 0.5 weight percent based on the total amount of base.

If the amount of polyvalent metal compound is less than 0.001 weight percent, it is difficult to form a crosslinked structure with an anionic group of the water-soluble copolymer (a). Consequently, it is difficult to impart characteristics such as adhesive strength, gel-formation, and shape-retaining property to the base. If the amount exceeds 3 weight percent, the adhesive force may be decreased because of an excessive crosslinking.

### (d) Water

The cataplasm base of the present invention is used for an aqueous composition. For example, purified water is generally used as water.

The specific amount of water depends on the amounts of components (a) to (c) described above, the amounts being determined according to the characteristics required for the cataplasm base. But the amount of water is generally 10 to 85 weight percent.

### (e) Water-soluble polyacrylic acid and/or salt thereof

In addition to the above indispensable components (a) to (d), the cataplasm base of the present invention preferably includes a water-soluble polyacrylic acid and/or a salt thereof serving as a thickening agent or a tackifier.

The usable salt of water-soluble polyacrylic acid includes salts in which all carboxyl groups or a part of the carboxyl groups of the polyacrylic acid forms a salt.

Examples of the salt of carboxyl groups of polyacrylic acid include alkali metal salts such as sodium salts and potassium salts; alkaline earth metal salts such as calcium salts and magnesium salts; and ammonium salts. Among these, sodium salts are preferable in view of safety and productivity.

Since preferably usable water-soluble polyacrylic acids and/or salts thereof are commercially available, these may be used without further treatment.

Examples of the commercially available water-soluble polyacrylic acid and/or a salt thereof include a partially neutralized powdery polyacrylic acid from Nihon Junyaku Co., Ltd., (trade name ARONVIS AH-105), a powdery sodium polyacrylate from Nihon Junyaku Co., Ltd., (trade name ARONVIS S), and an aqueous solution of polyacrylic acid from Nihon Junyaku Co., Ltd., (concentration: 20 weight percent, trade name JURIMER AC-10H), and a powdery crosslinked polyacrylic acid from Nihon Junyaku Co., Ltd., (trade name JUNLON PW-110). These may be used alone or in combination of two or more.

The amount is preferably 1 to 30 weight percent and more preferably 3 to 15 weight percent based on the total amount of base.

When the amount of water-soluble polyacrylic acid and/or a salt thereof is less than 1 weight percent, the contribution to the improvement of adhesive strength of the resultant base to the skin and the improvement of gel strength of the paste is small. When the amount exceeds 30 weight percent, the balance between the hardness and the adhesive force of the resultant base may not be kept.

### (f) Monohydric alcohol having 2 to 6 carbon atoms

The cataplasm base of the present invention may include a monohydric alcohol having 2 to 6 carbon atoms for some purposes.

This monohydric alcohol having 2 to 6 carbon atoms serves as a coolant in use of a cooling sheet, a solubilizing agent for evenly dissolving hydrophobic drugs and additives in the base, or a percutaneous absorption-promoting agent.

In view of safety and the effects, ethanol or isopropyl alcohol is preferably used as the monohydric alcohol having 2 to 6 carbon atoms. In particular, ethanol is preferable.

The amount is preferably 0.1 to 20 weight percent and more preferably 1 to 15 weight percent based on the total amount of base. At an amount exceeding 20 weight percent, a rash or an inflammation is readily caused.

### (g) Long-chain fatty acid and/or ester thereof

Also, the cataplasm base of the present invention may include a long-chain fatty acid and/or an ester thereof for any purpose. This long-chain fatty acid and/or an ester thereof is used as a solubilizing agent of hydrophobic additives and drugs mixed in the base or used to improve the percutaneous absorption property for medicinal components.

In view of safety and the effects, oleic acid, linoleic acid, linolenic acid, myristic acid, or an ester thereof is preferably used as the long-chain fatty acid and/or an ester thereof.

The amount is preferably 0.1 to 20 weight percent and more preferably 1 to 10 weight percent based on the total amount of base. When the amount is less than 0.1 weight percent, the contribution to the improvement of solubility of hydrophobic additives and drugs is small. In such a case, additives or drugs may be precipitated in the base or the percutaneous absorption property for drugs may be deteriorated.

The above-described components (e) to (g) may be used alone or in combination of two or more to add to the above indispensable components (a) to (d).

The combination includes the following:
(1) The combination of (e) a water-soluble polyacrylic acid and/or a salt thereof and (f) a monohydric alcohol having 2 to 6 carbon atoms;
(2) The combination of (e) a water-soluble polyacrylic acid and/or a salt thereof and (g) a long-chain fatty acid and/or an ester thereof;
(3) The combination of (f) a monohydric alcohol having 2 to 6 carbon atoms and (g) a long-chain fatty acid and/or an ester thereof; and
(4) The combination of (e) a water-soluble polyacrylic acid and/or a salt thereof, (f) a monohydric alcohol having 2 to 6 carbon atoms, and (g) a long-chain fatty acid and/or an ester thereof.

### (h) Organic acid having hydroxyl group or salt thereof

Furthermore, the cataplasm base of the present invention may include an organic acid having a hydroxyl group or a salt thereof, according to the desired characteristics.

This organic acid having a hydroxyl group or a salt thereof facilitates the dissolution of a slightly soluble metal compound by the hydroxyl group and a carboxyl group in the molecule so that the polyvalent metal compound, i.e., component (c) can easily release a polyvalent metal ion.

In view of skin irritation and safety, examples of the organic acid having a hydroxyl group or a salt thereof include tartaric acid, lactic acid, citric acid, malic acid, glycolic acid, gluconic acid, and alkali metal salts thereof. In particular, tartaric acid, citric acid, malic acid, and alkali metal salts thereof are preferable. For example, tartaric acid is preferable from the viewpoint that the combination with aluminum hydroxide or aluminum glycinate increases the curing rate of gel.

The amount is preferably 0.01 to 5 weight percent and more preferably 0.05 to 2 weight percent based on the total amount of base.

### (i) Drug or cosmetic component

The cataplasm base of the present invention is used in medicinal cataplasms or cosmetic cataplasms. The cataplasm base of the present invention may include a drug or a cosmetic component when used in such applications.

Examples of the drug or the cosmetic component include pharmaceutical products such as an anti-inflammatory agent, an analgesic, a bronchodilator, an antiasthmatic agent, and a cardiotonic; and cosmetic components such as a whitening agent.

Examples of the drug include indomethacin, ketoprofen, methyl salicylate, glycol salicylate, flurbiprofen, ibuprofen, suprofen, loxoprofen, ferbinac, piroxicam, meloxicam, tulobuterol, diphenhydramine, dibucaine, procaine, lidocaine, nitroglycerin, isosorbide nitrate, nicotine, and vitamins.

Examples of the whitening agent include ascorbic acid derivatives and salts thereof such as sodium ascorbate, magnesium L-ascorbyl phosphate, disodium L-ascorbyl sulfate, ascorbyl palmitate, ascorbyl dipalmitate, and stearyl ascorbate; Kojic acid and derivatives thereof; licorice extract; placenta extract; hydroquinone and derivatives thereof; arbutin; isoflavone derivatives; p-hydroxycinnamic acid derivatives; geraniin; gallic acid; cysteine, glutathione; colloidal sulfur; teprenone; 2-chromanone derivatives; and spiroether compounds.

The compounding ratio of the drugs or the whitening agents is preferably 0.1 to 20 weight percent based on the total amount of base.

### (j) Other component

In addition to the above components, in order to improve the quality in use, the cataplasm base of the present invention may include at least one other component selected from an antioxidant, an antiseptic, an emulsifier, and the like, which is an additive generally used in a cataplasm.

Examples of the other component include fragrant materials and coolants such as L-menthol, camphor, thymol, peppermint oil, castor oil, fennel oil, star anise oil, cinnamon oil, clove oil, thyme oil, turpentine oil, eucalyptus oil, lavender oil, lemon oil, orange oil, bergamot oil, and rose oil; warm feeling agents such as chili pepper extract and zanthoxylum extract; antioxidants such as dibutyl hydroxy toluene and butyl hydroxy anisole; antiseptics such as methyl parahydroxybenzoate and propyl parahydroxybenzoate; emulsifiers; and inorganic powders such as kaolin, titanium oxide, and silicic acid anhydride powders.

Specific examples of the compounding ratio of the fragrant materials, coolants, inorganic additives, and/or the like is generally 1 to 20 weight percent based on the total amount of cataplasm base.

### 2. Method for preparing cataplasm base

A method for preparing the cataplasm base of the present invention is not particularly limited.

In general, a water-soluble copolymer (a), a polyhydric alcohol (b), a polyvalent metal compound (c), components (e) to (g), component (j), and according to purpose, a drug or a cosmetic component (i) are added. An organic acid having a hydroxyl group or a salt thereof (h) is dissolved in water (d). These are mixed and kneaded until the mixture becomes uniform at room temperature. Subsequently, the mixture is deaerated under reduced pressure to prepare an aqueous swollen gel (aqueous gel paste).

### 3. Applications of cataplasm base

The cataplasm base of the present invention can be used for various applications.

The cataplasm base of the present invention can be used as a cataplasm base in many fields such as pharmaceutical products, e.g., an anti-inflammatory agent, a bronchodilator, an antiasthmatic agent, and a cardiotonic; cosmetics, e.g., a whitening agent; and pharmaceutical devices and daily commodities, e.g., a cold feeling agent, a cooling sheet, a warm feeling agent, and a portable body warmer.

### 4. Method of using cataplasm base and method for producing cataplasm

### 4.1 Skin cataplasm for drugs or cosmetics

Aqueous gel paste containing the cataplasm base of the present invention, a drug or a whitening agent for a desired purpose, and another component is applied by, for example, spreading on a backing composed of a non-woven fabric, a knitted cloth, a woven fabric, a paper sheet, a plastic film, or the like so as to form a layer.

Subsequently, according to need, the surface of the layer is covered with a separable film such as a polyethylene film, a sheet, or the like. Thus, cataplasms for drugs or cataplasms for cosmetics are produced. These are applied on the skin in use.

### 4.2 Pharmaceutical devices and daily commodities

When gel paste contains ethanol, moisture, or the like as an component and sufficiently holds the component, coldness or endothermic effect is provided by the heat of vaporization due to the vaporization of moisture from the base, thus providing coldness or cooling function to the skin. As in the above item 4.1, gel paste is applied on a backing such as a non-woven fabric or a plastic film to shape, thereby preparing a cold feeling agent, a cooling sheet, or the like.

### 4.3 Others

Furthermore, the cataplasm base of the present invention has heat resistance and keeps shape-retaining performance even under a certain level of high temperature (for example, at a temperature of about 50°C to about 60°C). Therefore, a warm feeling agent such as chili pepper extract is blended in gel paste as required. As in the above case, the gel paste is applied on a backing such as a non-woven fabric or a plastic film to shape, thereby preparing a cataplasm. For example, a keep-warm formed body such as a portable body warmer using iron oxide is bonded on this cataplasm with an appropriate adhesive or the like. Thus, an applying type keep-warmer (adhesive body warmer) can be produced.

### <Examples>

The present invention will now be more specifically described with reference to Examples and Comparative Examples. In examples described below, the symbol % represents weight percent unless otherwise stated.

### ⊙ Copolymer-Synthesizing Example 1

### <Synthesis of Copolymer 1>

50 weight percent aqueous solution (180.7 g, equivalent of 10 mole percent) of sodium 2-acrylamido-2-methylpropane sulfonate, 36 weight percent aqueous solution (309.0 g, equivalent of 30 mole percent) of sodium acrylate, acrylic acid (170.4 g, equivalent of 60 mole percent), and pure water (539.9 g) were mixed to prepare aqueous monomer solution (1.2 kg) having a monomer concentration of 31 weight percent.

The aqueous monomer solution was charged in a stainless steel Dewar vessel (i.e., reactor). Nitrogen bubbling was performed for 30 minutes while the temperature in the reactor was controlled to 10°C.

Subsequently, tert-butyl hydroperoxide (30 ppm, converted to the weight basis relative to the total amount of monomers, hereinafter the same as the above), sodium persulfate (200 ppm), and sodium erythorbate (30 ppm), which serve as polymerization initiators, were added to the solution. The solution was left to stand for 8 hours to perform heat insulating stationary redox polymerization.

After the 8-hour reaction, the resultant aqueous gel polymer was taken from the reactor. The polymer was charged in a chopper and was finely cut so as to form a ground meat shape.

The finely cut aqueous gel was dried in a hot-air dryer and was then crushed with a crusher. Furthermore, particles having a diameter exceeding 180 µm were removed with a 83-mesh standard sieve composed of a stainless steel (Japanese Industrial Standards (JIS) Z 8801, inner diameter: 200 mm, opening: 180 µm). Subsequently, the powder was dried for finishing. Thus, target Copolymer 1 was prepared in the form of fine powder.

### ⊙ Copolymer-Synthesizing Example 2

### <Synthesis of Copolymer 2>

50 weight percent aqueous solution (356.9 g, equivalent of 20 mole percent) of sodium 2-acrylamido-2-methylpropane sulfonate, 36 weight percent aqueous solution (203.4 g, equivalent of 20 mole percent) of sodium acrylate, acrylic acid (168.3 g, equivalent of 60 mole percent), and pure water (471.4 g) were mixed to prepare an aqueous monomer solution (1.2 kg) having a monomer concentration of 35 weight percent.

Other procedures were performed as in Synthesizing Example 1 to prepare target Copolymer 2 in the form of fine powder.

### ⊙ Copolymer-Synthesizing Example 3

### <Synthesis of Copolymer 3>

50 weight percent aqueous solution (507.4 g, equivalent of 30 mole percent) of sodium 2-acrylamido-2-methylpropane sulfonate, 36 weight percent aqueous solution (192.8 g, equivalent of 20 mole percent) of sodium acrylate, acrylic acid (132.9 g, equivalent of 50 mole percent), and pure water (366.9 g) were mixed to prepare an aqueous monomer solution (1.2 kg) having a monomer concentration of 38 weight percent.

Other procedures were performed as in Synthesizing Example 1 to prepare target Copolymer 3 in the form of fine powder.

### ⊙ Copolymer-Synthesizing Example 4

### <Synthesis of Copolymer 4>

50 weight percent aqueous solution (179.0 g, equivalent of 10 mole percent) of sodium 2-acrylamido-2-methylpropane sulfonate, 36 weight percent aqueous solution (204.1 g, equivalent of 20 mole percent) of sodium acrylate, acrylic acid (197.0 g, equivalent of 70 mole percent), and pure water (619.9 g) were mixed to prepare an aqueous monomer solution (1.2 kg) having a monomer concentration of 30 weight percent.

Other procedures were performed as in Synthesizing Example 1 to prepare target Copolymer 4 in the form of fine powder.

### ⊙ Copolymer-Synthesizing Example 5

### <Synthesis of Copolymer 5>

As in Synthesizing Example 1, 50 weight percent aqueous solution (180.7 g, equivalent of 10 mole percent) of sodium 2-acrylamido-2-methylpropane sulfonate, 36 weight percent aqueous solution (309.0 g, equivalent of 30 mole percent) of sodium acrylate, acrylic acid (170.4 g, equivalent of 60 mole percent), and pure water (539.9 g) were mixed to prepare an aqueous monomer solution (1.2 kg) having a monomer concentration of 31 weight percent. The solution was polymerized, the resultant polymer was then finely cut, and dried as in Synthesizing Example 1.

Subsequently, the polymer was crushed with a crusher under a different condition such that the finely powdered copolymer has a smaller particle diameter. Other procedures were performed as in Synthesizing Example 1 to prepare target Copolymer 5 in the form of fine powder.

### ⊙ Copolymer-Synthesizing Example 6

### <Synthesis of Copolymer 6>

Polymerization was performed, the resultant polymer was then finely cut, and dried as in Synthesizing Example 1. Subsequently, the polymer was crushed with a crusher under a different condition such that the finely powdered copolymer has a larger particle diameter. Particles having a diameter exceeding 180 µm were not removed. Other procedures were performed as in Synthesizing Example 1 to prepare target Copolymer 6 in the form of fine powder.

Additionally, a part of the sample was screened with an 83-mesh standard sieve composed of a stainless steel. According to the result, the ratio of particles having a particle diameter exceeding 180 µm was 90 weight percent relative to the total amount of the copolymer.

### ⊙ Copolymer-Synthesizing Example 7

### <Synthesis of Copolymer 7>

Polymerization was performed, the resultant polymer was then finely cut, and dried as in Synthesizing Example 5. Subsequently, the polymer was crushed with a crusher under a different condition such that the finely powdered copolymer has a smaller particle diameter as in Synthesizing Example 5. Particles having a diameter exceeding 75 µm were then removed with a 200-mesh standard sieve composed of a stainless steel (JIS Z 8801, inner diameter: 200 mm, opening: 75 µm).

Other procedures were performed as in Synthesizing Example 5 to prepare target Copolymer 7 in the form of fine powder.

### ⊙ Comparative Copolymer-Synthesizing Example 1

### <Synthesis of Comparative Copolymer 1>

36 weight percent aqueous solution (566.2 g, equivalent of 50 mole percent) of sodium acrylate, acrylic acid (156.2 g, equivalent of 50 mole percent), and pure water (477.6 g) were mixed to prepare an aqueous monomer solution (1.2 kg) having a monomer concentration of 30 weight percent.

Other procedures were performed as in Synthesizing Example 1 to prepare target powdery Comparative Copolymer 1.

### ⊙ Comparative Copolymer-Synthesizing Example 2

### <Synthesis of Comparative Copolymer 2>

50 weight percent aqueous solution (775.1 g, equivalent of 50 mole percent) of sodium 2-acrylamido-2-methylpropane sulfonate, 36 weight percent aqueous solution (220.9 g, equivalent of 25 mole percent) of sodium acrylate, acrylic acid (60.9 g, equivalent of 25 mole percent), and pure water (143.1 g) were mixed to prepare an aqueous monomer solution (1.2 kg) having a monomer concentration of 44 weight percent.

Other procedures were performed as in Synthesizing Example 1 to prepare target powdery Comparative Copolymer 2.

### ⊙ Comparative Copolymer-Synthesizing Example 3

### <Synthesis of Comparative Copolymer 3>

To the same aqueous monomer solution as that in Synthesizing Example 1, tert-butyl hydroperoxide (300 ppm), sodium persulfate (200 ppm), which serve as polymerization initiators, and sodium erythorbate (300 ppm), and N,N'-methylenebisacrylamide (150 ppm), which serves as a bifunctional crosslinking agent, were added.

Other procedures were performed as in Synthesizing Example 1 to prepare target powdery Comparative Copolymer 3.

### ⊙ Comparative Copolymer-Synthesizing Example 4

### <Synthesis of Comparative Copolymer 4>

36 weight percent aqueous solution (449.8 g, equivalent of 40 mole percent) of sodium acrylate, acrylic acid (186.1 g, equivalent of 60 mole percent), and pure water (564.1 g) were mixed to prepare an aqueous monomer solution (1.2 kg) having a monomer concentration of 29 weight percent. Other procedures were performed as in Synthesizing Example 1 to prepare target powdery Comparative Copolymer 4.

Subsequently, physical properties of Copolymers 1 to 7 prepared in Synthesizing Examples 1 to 7 and Comparative Copolymers 1 to 4 prepared in Comparative Synthesizing Examples 1 to 4 were evaluated according to the following test methods.

Table 1 shows the results.

### [Test methods]

### (1) Solid content

In an aluminum cup, 1.00 g of each copolymer prepared in Synthesizing Examples 1 to 7 and Comparative Synthesizing Examples 1 to 4 was weighed. The copolymer was heated in a dryer at a temperature of 105°C for 3 hours. The solid content was determined from the loss in weight by heating.

### (2) Viscosity of 0.2 weight percent aqueous solution

Each copolymer (1.00 g) prepared in Synthesizing Examples 1 to 7 and Comparative Synthesizing Examples 1 to 4 was added to pure water (500 mL) and the mixture was stirred for 3 hours to sufficiently dissolve. Thus, aqueous copolymer solutions having a concentration of 0.2 weight percent were prepared.

The viscosity of each aqueous copolymer solution was measured with a B-type viscometer (from Tokimec Inc., type: BM type) at a temperature of 30°C and at a rotational speed of rotor of 30 rpm.

### (3) Weight-average molecular weight

The molecular weight of each copolymer prepared in Synthesizing Examples 1 to 7 and Comparative Synthesizing Examples 1 to 4 was measured by an aqueous GPC method.

With respect to the eluent, an aqueous solution containing sodium sulfate (1.33 g/L) and sodium hydroxide (0.33 g/L) as the solutes was used. The weight-average molecular weight was calculated from a calibration curve prepared with a polyethylene oxide as a standard material.

### (4) Content of unreacted monomers (remaining monomers)

Each copolymer (1.00 g) prepared in Synthesizing Examples 1 to 7 and Comparative Synthesizing Examples 1 to 4 was added to an 80 volume percent aqueous solution of acetonitrile (20 mL) and the mixture was stirred for 1 hour. The mixture was then left to stand for 1 hour to extract the unreacted monomers. The supernatant was collected to perform a measurement by high performance liquid chromatography.

An HPLC packed column #3056 from Hitachi, Ltd. was used as a separation column. 0.1 weight percent phosphate buffer was used as the eluent. The content of unreacted monomers was calculated in terms of weight percent relative to the total amount of copolymer.

### (5) Content of particles having particle diameter of 180 µm or more

One hundred grams of each copolymer prepared in Synthesizing Examples 1 to 7 and Comparative Synthesizing Examples 1 to 4 was weighed. The copolymer was screened with an 83-mesh standard sieve composed of stainless steel (JIS Z 8801, inner diameter: 200 mm, opening: 180 µm). The content of particles having a particle diameter of 180 µm or more was calculated in terms of weight percent relative to the total amount of copolymer.

### (6) Content of particles having particle diameter of 75 µm or less

One hundred grams of each copolymer prepared in Synthesizing Examples 1 to 7 and Comparative Synthesizing Examples 1 to 4 was weighed. The copolymer was screened with a 200-mesh standard sieve composed of stainless steel (JIS Z 8801, inner diameter: 200 mm, opening: 75 µm). The content of particles having a particle diameter of 75 µm or less was calculated in terms of weight percent relative to the total amount of copolymer.

**Table 1-1**

| | Monomer composition ATBS-Na/ANa/AA [mol%] | Neutralization ratio [mol%] | Solid content [wt%] | Viscosity of 0.2% aq. sol, [mPa·s] | Remaining monomer [wt%] |
|---|---|---|---|---|---|
| Copolymer 1 | 10/30/60 | 40 | 96.3 | 564 | 0.14 |
| Copolymer 2 | 20/20/60 | 40 | 96.7 | 505 | 0.16 |
| Copolymer 3 | 30/20/50 | 50 | 96.5 | 484 | 0.17 |
| Copolymer 4 | 10/20/70 | 30 | 96.2 | 492 | 0.20 |
| Copolymer 5 | 10/30/60 | 40 | 96.4 | 480 | 0.16 |
| Copolymer 6 | 10/30/60 | 40 | 96.2 | 605 | 0.14 |
| Copolymer 7 | 10/30/60 | 40 | 96.8 | 391 | 0.18 |
| Comparative Copolymer 1 | -/50/50 | 50 | 96.8 | 611 | 0.09 |
| Comparative Copolymer 2 | 50/25/25 | 75 | 96.6 | 440 | 0.17 |
| Comparative Copolymer 3 | 10/30/60 | 40 | 96.3 | 545 | 0.15 |
| Comparative Copolymer 4 | -/40/60 | 40 | 96.5 | 560 | 0.16 |

The abbreviations in the table represent the following.
ATBS-Na: sodium 2-acrylamido-2-methylpropanesulfonate
A-Na: sodium acrylate
AA: acrylic acid
ATBS is a registered trademark from Toagosei Co., Ltd.

**Table 1-2**

| | Weight-average molecular weight | Particle size[wt%] | | Indissolubility [ml] |
|---|---|---|---|---|
| | | >180µm | <75µm | |
| Copolymer 1 | 7,800,000 | 0 | 23 | 0 |
| Copolymer 2 | 8,000,000 | 0 | 25 | 0 |
| Copolymer 3 | 8,200,000 | 0 | 32 | 0 |
| Copolymer 4 | 7,700,000 | 0 | 21 | 0 |
| Copolymer 5 | 7,650,000 | 0 | 60 | 0 |
| Copolymer 6 | 7,900,000 | 90 | ≦ 1 | 0 |
| Copolymer 7 | 7,400,000 | 0 | 100 | 0 |
| Comparative Copolymer 1 | 8,700,000 | 0 | 22 | 0 |
| Comparative Copolymer 2 | 8,500,000 | 0 | 28 | 0 |
| Comparative Copolymer 3 | immeasurable | 0 | 26 | 250 |
| Comparative Copolymer 4 | 8,000,000 | 0 | 23 | 0 |

### ⊙ Examples 1 to 11 and Comparative Examples 1 to 4

### [Method for producing cataplasm]

### (1) Preparation of cataplasm base

Components shown in Tables 2-1, 2-2, and 2-3 were kneaded with a mixing apparatus (i.e., kneader) in the ratio (parts by weight) shown in Tables 2-1, 2-2, and 2-3 at room temperature until each mixture becomes uniform. The mixture is then deaerated under reduced pressure to prepare a cataplasm base, i.e., aqueous gel paste.

### (2) Production of cataplasm

Each aqueous gel paste prepared in the above (1) was applied on a stretch non-woven fabric (non-woven polyester fabric having a weight of 105 g/m²) so as to have thickness of about 1 mm (10 g/100 cm²). The paste was covered with a release film to produce a cataplasm.

### (3) Packaging and shaping

Each cataplasm prepared in the above (2) was sandwiched with two foil laminate films from the directions of both surfaces of the cataplasm. The periphery of the films was then packaged with air tightness by heat sealing.

The resultant cataplasm was stored and shaped at a temperature of 25°C and with a relative humidity of 50% for 240 hours.

### [Method for evaluating cataplasm]

Each shaped cataplasm was taken from the airtight package material and was evaluated with respect to the following items.
(1) Adhesive strength: The cataplasms were applied on arms of at least 5 persons. The time taken for naturally detaching was measured. The adhesive strength was evaluated from the average based on the following criterion.
   ⊙: The average time taken for naturally detaching was 5 hours or more.
   ○: The average time taken for naturally detaching was 3 hours or more and less than 5 hours.
   Δ: The average time taken for naturally detaching was less than 3 hours.
(2) Seepage resistance: The reverse face of non-woven polyester fabric was visually observed. The seepage resistance was evaluated based on the following criterion.
   ○: No seepage was observed.
   Δ: The seepage was partially observed.
   ×: The seepage was observed overall.
(3) Paste residue: Each cataplasm was applied on the arm and then lightly pressed with the palm. Subsequently, the cataplasm was immediately detached. The presence of paste remaining on the arm was visually observed. The paste residue was evaluated based on the following criterion.
   ○: The paste did not remain on the arm at all.
   Δ: A part of the paste remained on the arm.
(4) Hardness: Each cataplasm was applied on the outside of the elbow bent by about 90° and then lightly pressed with the palm. Subsequently, the elbow was straightened and the adhesion state was visually observed.
   ⊙: No bubbled portion was observed and the cataplasm was satisfactorily adhered.
   ○: Although a bubbled portion was partially observed, the cataplasm was stably adhered overall.
   Δ: Bubbled portion was observed over the entire elbow.
(5) Finished state of paste surface: The release film was removed and the finished state of the paste surface of each cataplasm was visually inspected. The finished state of the paste surface was evaluated based on the following criterion.
   ○: The surface was smooth and flat. The finishing was satisfactory.
   Δ: Irregularities were slightly observed on the surface.

The appellations in the table represent the following.
ARONVIS AH-105: partially neutralized powdery polyacrylic acid (from Nihon Junyaku Co., Ltd.)
JURIMER AC-10H: aqueous solution of polyacrylic acid having a concentration of 20 weight percent (from Nihon Junyaku Co., Ltd.)
ARONVIS AH-105 and JURIMER AC-10H are registered trademarks from Nihon Junyaku Co., Ltd.

**Table 2-1**

| Cataplasm base(ppw) | Example | | | | |
|---|---|---|---|---|---|
| | 1 | 2 | 3 | 4 | 5 |
| (a)Copolymer | [1] | [2] | [3] | [4] | [5] |
| | 8 | 8 | 8 | 8 | 8 |
| (b) glycerin | 10 | 10 | 10 | 10 | 10 |
| (c)aluminum hydroxide | 0.3 | | 0.3 | | 0.3 |
| aluminum glycinate | | 0.3 | | 0.3 | |
| (d)purified water | 61. 1 | 80. 6 | 67. 1 | 80. 6 | 78.1 |
| (f)ethanol | 20 | | 10 | | |
| (g)oleic acid | | | 2 | | |
| myristic acid | | | | | 2 |
| (h) tartaric acid | 0.1 | 0.1 | 0.1 | 0.1 | 0.1 |
| (i)indomethacin | 0.5 | | | 0.5 | |
| ketoprofen | | 0.5 | | | 0.5 |
| methyl salicylate | | | 1.5 | | |
| (j)peppermint oil | | 0.5 | | 0.5 | |
| L-menthol | | | 1 | | 1 |
| Total | 1 00 | 1 00 | 100 | 100 | 100 |
| Water content (wt%) | 61.1 | 80.6 | 67.1 | 80.6 | 78.1 |
| Property | | | | | |
| Adhesiveness | ⊚ | ⊚ | ⊚ | ⊚ | ⊚ |
| Seepage resistance | ○ | ○ | ○ | ○ | ○ |
| Paste residue | ○ | ○ | ○ | ○ | ○ |
| Hardness | ⊚ | ○ | ○ | ⊚ | ⊚ |
| Finished state of paste surface | ○ | ○ | ○ | ○ | ○ |

**Table 2-2**

| Cataplasm base(ppw) | Example | | | | | |
|---|---|---|---|---|---|---|
| | 6 | 7 | 8 | 9 | 10 | 11 |
| (a)Copolymer | [1] | [1] | [6] | [7] | [1] | [1] |
| | 8 | 8 | 8 | 8 | 10 | 8 |
| (b)glycerin | 12 | 12 | 12 | 12 | 30 | 10 |
| (c) aluminum hydroxide | 0.3 | | 0.3 | | 0.5 | 0.3 |
| Aluminum glycinate | | 0.3 | | 0.3 | | |
| (d) purified water | 55. 6 | 66. 1 | 76. 6 | 55. 1 | 56. 2 | 81. 6 |
| (e) ARONVIS AH-105 | 2 | | 2 | | | |
| JURIMER AC-10H | | 10 | | 10 | | |
| (f)ethanol | 20 | | | 10 | | |
| (g)oleic acid | | 2 | | | | |
| Myristic acid | | | | 2 | | |
| (h) tartaric acid | 0.1 | 0.1 | 0.1 | 0.1 | 0.3 | 0.1 |
| (i) indomethacin | | 0.5 | | | | |
| Ketoprofen | | | 0.5 | | | |
| methyl salicylate | 1.5 | | | 1. 5 | | |
| Magnesium L-ascorbyl phosphate | | | | | 3 | |
| (j)peppermint oil | 0. 5 | | 0.5 | | | |
| L-menthol | | 1 | | 1 | | |
| Total | 100 | 100 | 100 | 100 | 100 | 100 |
| Water content (wt%) | 55. 6 | 74. 1 | 76. 6 | 63. 1 | 56. 2 | 81.6 |
| Property | | | | | | |
| Adhesiveness | ⊚ | ⊚ | ⊚ | ⊚ | ⊚ | ⊚ |
| Seepage resistance | ○ | ○ | ○ | ○ | ○ | ○ |
| Paste residue | ○ | ○ | ○ | ○ | ○ | ○ |
| Hardness | ⊚ | ⊚ | ⊚ | ⊚ | ⊚ | ⊚ |
| Finished state of Paste state | ○ | ○ | Δ | Δ | ○ | ○ |

**Table 2-3**

| | Comparative Example | | | |
|---|---|---|---|---|
| Cataplasm base(ppw) | 1 | 2 | 3 | 4 |
| (a) Comparative copolymer | (1) | (2) | (3) | (4) |
| | 8 | 8 | 8 | 10 |
| (b)glycerin | 10 | 10 | 10 | 30 |
| (c) aluminum hydroxide | 0.3 | | 0.3 | 0.5 |
| aluminum glycinate | | 0.3 | | |
| (d)purified water | 61.1 | 80. 6 | 67.1 | 56. 2 |
| (f) ethanol | 20 | | 10 | |
| (g) oleic acid | | | 2 | |
| (h) tartaric acid | 0.1 | 0.1 | 0.1 | 0.3 |
| (i)indomethacin | 0.5 | | | |
| ketoprofen | | 0.5 | | |
| Methyl salicylate | | | 1.5 | |
| magnesium L-ascorbyl phosphate | | | | 3 |
| (j)peppermint oil | | 0.5 | | |
| L-menthol | | | 1 | |
| Total | 1 00 | 100 | 100 | 100 |
| Water content (wt%) | 61. 1 | 80. 6 | 67. 1 | 56. 2 |
| Property | | | | |
| Adhesiveness | Δ | Δ | Δ | Δ |
| Seepage resistance | Δ | ○ | ○ | × |
| Paste residue | Δ | ○ | ○ | ○ |
| Hardness | Δ | Δ | Δ | ○ |
| Finished state of Paste state | Δ | ○ | Δ | Δ |

As is apparent from Tables 2-1, 2-2, and 2-3, cataplasms in Examples showed satisfactory results, compared with those in Comparative Examples.

That is, in Examples 1 to 11, the adhesive strength of each cataplasm was excellent. In addition, as shown in Example 1 and Examples 4 to 11, when the ratio of 2-acrylamido-2-methylpropane sulfonic acid in the water-soluble copolymer (a) of the base is as low as 10 mole percent, higher hardness of the gel paste was ensured.

As shown in Examples 6 to 9, which included a water-soluble polyacrylic acid and a salt thereof (e) in the base, both the adhesive strength and the hardness were satisfactory.

In Examples 8 and 9, which included a large amount of coarse particles of the water-soluble copolymer (a) having a particle diameter of at least 180 µm or a large amount of fine particles of the water-soluble copolymer (a) having a particle diameter of up to 75 µm, irregularities were slightly observed on the surface of the paste. Except that, in all Examples, cataplasms that did not cause seepage and paste residue and that had a satisfactory finished surface of paste were produced.

In contrast, in Comparative Example 1 using Comparative Copolymer 1, all the evaluated items, i.e., the adhesive strength, the seepage resistance, the paste residue, the hardness, and the surface finishing were not satisfactory. Also, in Comparative Example 2 using Comparative Copolymer 2, the adhesive strength and the hardness were not satisfactory.

Furthermore, in Comparative Example 3 using Comparative Copolymer 3 in which a polyfunctional crosslinking agent was added in polymerization of the water-soluble copolymer (a), the adhesive strength, the hardness, and the surface finishing were not satisfactory. Also, in Comparative Example 4 using Comparative Copolymer 4, which did not include 2-acrylamido-2-methylpropane sulfonic acid and had the same neutralization ratio as that of Copolymer 1, the adhesive strength and the surface finishing were not satisfactory.

In particular, since magnesium L-ascorbyl phosphate, which was a whitening agent, was blended, the shape-retaining property and the curing property were significantly deteriorated. As a result, seepage was generated on the overall non-woven fabric.

### ⊙ Examples 12 to 14 and Comparative Examples 5 to 7

### [Preparation of adhesive body warmer]

First, a shaped cataplasm and a commercially available disposable body warmer were taken from the airtight packages. The surface of the non-woven fabric of the cataplasm was bonded with the disposable body warmer with an adhesive to prepare an adhesive body warmer for evaluation.

### [Method for evaluating adhesive body warmer]

Each adhesive body warmer prepared as described above was evaluated with respect to the following items.
(1) Initial adhesive strength: Adhesive body warmers immediately after preparation were applied on the lower back of at least 5 persons. Each adhesive body warmer was immediately detached. The detachment strength at the time was evaluated with the following three levels.
   ⊚: The adhesive body warmer had a sufficiently stable adhesive strength.
   ○: The adhesive strength was not sufficient but the adhesive body warmer had an adhesive strength to the extent that the body warmer was not naturally detached.
   Δ: The adhesive body warmer had an insufficient adhesive strength and was not applied successfully.
(2) Heat resistant adhesive strength: Adhesive body warmers immediately after preparation were applied on the lower back of at least 5 persons. The time taken for naturally detaching was measured. The heat resistant adhesive strength under warming state was evaluated from the average based on the following criterion.
   ⊚: The average time taken for naturally detaching was 3 hours or more.
   ○: The average time taken for naturally detaching was 1 hour or more and less than 3 hours.
   Δ: The average time taken for naturally detaching was less than 1 hour.
(3) Heat resistant paste residual property: Adhesive body warmers immediately after preparation were applied on the lower back of at least 5 persons. Each of the adhesive body warmers was fixed with a supporter so as not to be detached. Thus, the adhesive body warmer was applied for 5 hours. Subsequently, the adhesive body warmer was detached and the presence of paste remaining on the skin was visually observed. The heat resistant paste residual property under warming state was evaluated based on the following criterion.
   ○: The paste did not remain on the skin at all.
   Δ: A part of the paste remained on the skin.
(4) Sweat-resistant adhesive strength: Adhesive body warmers immediately after preparation were applied on the lower back of at least 5 persons. Each of the adhesive body warmers was fixed with a supporter so as not to be detached. In this state, the persons had a little exercise for 30 minutes to perspire. Subsequently, the adhesive body warmers were detached. The detachment strength at the time was evaluated with the following four levels.
   ⊚: The adhesive body warmer had a sufficiently stable adhesive strength.
   ○: The adhesive strength was not sufficient but the adhesive body warmer had an adhesive strength to the extent that the body warmer was not detached even when the test subject had a little exercise.
   Δ: The strength was significantly decreased and the adhesive body warmer had an adhesive strength to the extent that the body warmer was detached when the persons had a little exercise.
   ×: The adhesive body warmer was barely applied and the adhesive body warmer had an adhesive strength to the extent that the body warmer was detached even when the persons subject stood still.
(5) Readhesion property: After the above sweat-resistant adhesive strength was evaluated, the sweat on the applied portion of the lower back was wiped off and each adhesive body warmer after evaluation was applied on the lower back again. The adhesive body warmer was immediately detached and the detachment strength at the time was evaluated with the following four levels.
   Ⓞ: The adhesive body warmer had a sufficiently stable adhesive strength.
   ○: The adhesive strength was not sufficient but the adhesive body warmer had an adhesive strength to the extent that the body warmer was not detached even when the persons had a little exercise.
   Δ: The strength was significantly decreased and the adhesive body warmer had an adhesive strength to the extent that the body warmer was detached when the persons had a little exercise.
   ×: The adhesive body warmer was barely applied and the adhesive body warmer had an adhesive strength to the extent that the body warmer was detached even when the persons stood still.

**Table 3**

| Cataplasm base (ppw) | Example | | | Comparative Example | | |
|---|---|---|---|---|---|---|
| | 12 | 13 | 14 | 5 | 6 | 7 |
| (a)Copolymer | [1] | [1] | [1] | | | |
| Comparative copolymer | | | | (4) | (4) | (4) |
| | 10 | 10 | 10 | 10 | 10 | 10 |
| (b) glycerin | 30 | 30 | 30 | 30 | 30 | 30 |
| (c)aluminum hydroxide | 0.5 | 0.5 | 0.5 | 0.5 | 0.5 | 0.5 |
| (d)purified water | 59.2 | 56.2 | 48.2 | 59.2 | 56.2 | 48.2 |
| (e)ARONVIS AH-105 | | 2 | | | 2 | |
| JURIMER AC-10H | | | 10 | | | 10 |
| (h) tartaric acid | 0.3 | 0.3 | 0.3 | 0.3 | 0.3 | 0.3 |
| (j)chili pepper extract | | 1 | | | | |
| zanthoxylum extract | | | 1 | | | 1 |
| Total | 100 | 100 | 100 | 100 | 100 | 100 |
| Water content (wt%) | 59.2 | 56.2 | 56.2 | 59.2 | 56.2 | 56.2 |
| Property | | | | | | |
| Initial adhesive strength | ○ | ⊚ | ⊚ | ○ | ○ | ○ |
| Heat resistance adhesive strength | ⊚ | ⊚ | ⊚ | Δ | Δ | Δ |
| Heat resistant paste residual property | ○ | ○ | ○ | Δ | Δ | Δ |
| Sweat-resistant Adhesive strength | ⊚ | ⊚ | ⊚ | × | × | Δ |
| Readhesion property | ○ | ○ | ⊚ | × | × | Δ |

As shown in Table 3, adhesive body warmers in Examples showed satisfactory results, compared with those in Comparative Examples.

That is, in Examples 12 to 14, the adhesive strength of each adhesive body warmer was excellent. As shown in Example 12, the use of Copolymer 1 provided excellent adhesive strength, compared with Comparative Example 5 using the partially neutralized polyacrylic acid (Comparative Copolymer 4), which did not include 2-acrylamido-2-methylpropane sulfonic acid and had the same neutralization ratio as that of Copolymer 1. In particular, the heat resistant adhesive strength, the sweat-resistant adhesive strength, and the readhesion property were excellent to satisfy the adhesion performance.

In addition, combining the partially neutralized powdery polyacrylic acid (ARONVIS AH-105) as the water-soluble polyacrylic acid and a salt thereof (e) improved the initial adhesive strength (Example 13).

Furthermore, combining the aqueous solution of polyacrylic acid having a concentration of 20 weight percent (JURIMER AC-10H) as the water-soluble polyacrylic acid (or a salt thereof) (e) improved the initial adhesive strength and the readhesion property (Example 14).

In contrast, in Comparative Examples 5 to 7 using the partially neutralized polyacrylic acid (Comparative Copolymer 4) as the water-soluble copolymer (a), which did not include 2-acrylamido-2-methylpropane sulfonic acid as a comonomer, a certain degree of initial adhesive strength was provided. However, other all evaluated items, that is, the heat resistant adhesive strength, the heat resistant paste residue, the sweat-resistant adhesive strength, and the readhesion property were not satisfactory.

### <Industrial Applicability>

A cataplasm base of the present invention and a cataplasm using the same can contain a significantly large amount of moisture. Therefore, when the cataplasm base of the present invention or the cataplasm using the same is applied on the skin or the like, a cooling effect can be maintained for a long time, and in addition, an excellent percutaneous absorption property of drugs can be provided.

Despite high water content, the cataplasm base of the present invention and the cataplasm using the same have excellent shape-retaining property and heat resistance. Therefore, paste does not seep from a backing composed of a non-woven fabric or the like. Furthermore, the cataplasm base of the present invention and the cataplasm using the same have excellent adhesive strength to the skin or the like, and are difficult to be detached when applied, and have appropriate hardness. When the cataplasm base of the present invention and the cataplasm using the same are detached from the skin, the paste does not remain on the skin. The cataplasm base of the present invention and the cataplasm using the same can be applied for a long time.

Furthermore, since the cataplasm base is prepared after the water-soluble copolymer is prepared, the content of unreacted remaining monomers, which are impurities, is low. Thus, skin problems such as erythema, skin fit, and a rash can be prevented.

As described above, the cataplasm base of the present invention and the cataplasm using the base can be widely applied to dermatological cataplasms for pharmaceutical products or cosmetics, pharmaceutical devices, and daily commodities.

## Claims

1. A cataplasm comprising a backing and an aqueous gel paste located on the backing, the aqueous gel paste comprising the following components:
(a) a water-soluble copolymer;
(b) a polyhydric alcohol;
(c) a polyvalent metal compound; and
(d) water;
wherein the water-soluble copolymer is in the form of fine powder and includes 1 to 30 mole percent of 2-acrylamido-2-methylpropane sulfonic acid and 70 to 99 mole percent of acrylic acid as monomer units, wherein 20 to 60 mole percent of the acids is neutralized, and wherein the content of unreacted monomers in the water-soluble copolymer is 0.5 weight percent or less.

2. The cataplasm according to claim 1, wherein each of the aqueous gel paste components has the following blending ratio based on the total amount of the aqueous gel paste:
(a) the water-soluble copolymer : 1 to 30 weight percent;
(b) the polyhydric alcohol : 1 to 50 weight percent;
(c) the polyvalent metal compound : 0.001 to 3 weight percent; and
(d) water : balance.

3. The cataplasm according to claim 1, wherein the water-soluble copolymer in the form of fine powder comprises at least 90 weight percent of particles having a particle diameter of 180 µm or less, and in addition, the ratio of particles having a particle diameter of 75 µm or less is 80 weight percent or less.

4. The cataplasm according to claim 1, the aqueous gel paste further comprising at least one component selected from the following:
(e) a water-soluble polyacrylic acid and/or a salt thereof;
(f) a monohydric lower alcohol having 2 to 6 carbon atoms;
(g) a long-chain fatty acid and/or an ester thereof; and
(h) an organic acid having a hydroxyl group or a salt thereof.

5. The cataplasm according to claim 4, wherein the monohydric lower alcohol is ethanol or isopropyl alcohol.

6. The cataplasm according to claim 4, wherein the organic acid having a hydroxyl group or a salt thereof is tartaric acid or a salt thereof.

7. The cataplasm according to claim 1, wherein the aqueous gel paste contains a drug or a whitening agent.

## Patentansprüche

1. Kataplasma umfassend einen Träger und eine wässrige Gelpaste, die sich auf dem Träger befindet, wobei die wässrige Gelpaste folgende Komponenten umfasst:
(a) ein wasserlösliches Copolymer;
(b) einen mehrwertigen Alkohol:
(c) eine mehrwertige Metallverbindung; und
(d) Wasser;
wobei das wasserlösliche Copolymer in Form von weißem Pulver vorliegt und 1 bis 30 Molprozent 2-Acrylamido-2-methylpropansulfonsäure und 70 bis 99 Molprozent Acrylsäure als Monomereinheiten umfasst, wobei 20 bis 60 Molprozent der Säuren neutralisiert werden, und wobei der Gehalt an unreagierten Monomeren in dem wasserlöslichen Copolymer 0,5 Gewichtsprozent oder weniger beträgt.

2. Kataplasma nach Anspruch 1, wobei jede der Komponenten der wässrigen Gelpaste folgendes Mischungsverhältnis, auf die Gesamtmenge der wässrigen Gelpaste bezogen, aufweist:
(a) das wasserlösliche Copolymer: 1 bis 30 Gewichtsprozent;
(b) der mehrwertige Alkohol: 1 bis 50 Gewichtsprozent;
(c) die mehrwertige Metallverbindung: 0,001 bis 3 Gewichtsprozent; und
(d) Wasser: den Rest bildet.

3. Kataplasma nach Anspruch 1, wobei das wasserlösliche Copolymer in Form von feinem Pulver vorliegt, das mindestens 90 Gewichtsprozent Teilchen umfasst, die eine Teilchengröße von 180 µm oder weniger aufweisen und außerdem das Verhältnis von Teilchen, die einen Teilchendurchmesser von 75 µm oder weniger aufweisen, 80 Gewichtsprozent oder weniger beträgt.

4. Kataplasma nach Anspruch 1, wobei die wässrige Gelpaste des Weiteren mindestens eine Komponente umfasst ausgewählt unter den Folgenden:
(e) einer wasserlöslichen Polyacrylsäure und/oder einem Salz derselben;
(f) einem einwertigen niederen Alkohol, der 2 bis 6 Kohlenstoffatome aufweist;
(g) einer langkettigen Fettsäure und/oder einem Ester derselben; und
(h) einer organischen Säure, die eine Hydroxylgruppe aufweist, oder einem Salz derselben.

5. Kataplasma nach Anspruch 4, wobei der einwertige niedere Alkohol Ethanol oder Isopropylalkohol ist.

6. Kataplasma nach Anspruch 4, wobei die organische Säure, die eine Hydroxylgruppe aufweist, oder ein Salz derselben Tartarsäure oder ein Salz derselben ist.

7. Kataplasma nach Anspruch 1, wobei die wässrige Gelpaste ein Arzneimittel oder ein Weißungsmittel enthält.

## Revendications

1. Cataplasme comprenant un support et une pâte de type gel aqueux située sur le support, la pâte de type gel aqueux comprenant les composants suivants:
(a) un copolymère soluble dans l'eau;
(b) un alcool polyhydrique;
(c) un composé de métal polyvalent; et
(d) de l'eau;
dans lequel le copolymère soluble dans l'eau se présente sous la forme d'une poudre fine et inclut 1 à 30 pour cent en mole d'acide 2-acrylamido-2-méthylpropane sulfonique et 70 à 99 pour cent en mole d'acide acrylique comme motifs monomères, dans lequel 20 à 60 pour cent en mole des acides est neutralisé, et dans lequel la teneur des monomères n'ayant pas réagi dans le copolymère soluble dans l'eau est de 0,5 pour cent en poids ou moins.

2. Cataplasme selon la revendication 1, dans lequel chacun des composants de la pâte de type gel aqueux a le rapport suivant de mélange basé sur la quantité totale de la pâte de type gel aqueux:
(a) le copolymère soluble dans l'eau: 1 à 30 pour cent en poids;
(b) l'alcool polyhydrique: 1 à 50 pour cent en poids;
(c) le composé de métal polyvalent: 0,001 à 3 pour cent en poids; et
(d) l'eau: le reste.

3. Cataplasme selon la revendication 1, dans lequel le copolymère soluble dans l'eau sous la forme d'une poudre fine comprend au moins 90 pour cent en poids de particules ayant un diamètre de particule de 180 µm ou moins, et de plus, le rapport des particules ayant un diamètre de particule de 75 µm ou moins est de 80 pour cent en poids ou moins.

4. Cataplasme selon la revendication 1, la pâte de type gel aqueux comprenant en outre au moins un composant choisi parmi les suivants:
(e) un acide polyacrylique soluble dans l'eau et/ou un sel de celui-ci;
(f) un alcool monohydrique inférieur ayant 2 à 6 atomes de carbone;
(g) un acide gras à longue chaîne et/ou un ester de celui-ci; et
(h) un acide organique ayant un groupe hydroxyle ou un sel de celui-ci.

5. Cataplasme selon la revendication 4, dans lequel l'alcool monohydrique inférieur est l'éthanol ou l'alcool d'isopropyle.

6. Cataplasme selon la revendication 4, dans lequel l'acide organique ayant un groupe hydroxyle ou un sel de celui-ci est l'acide tartarique ou un sel de celui-ci.

7. Cataplasme selon la revendication 1, dans lequel la pâte de type gel aqueux contient un médicament ou un agent blanchissant.
